# EUROPEAN PATENT SPECIFICATION

(11) **EP 1 978 928 B1**
(45) Date of publication and mention of the grant of the patent: **31.03.2010**
(21) Application number: 07703695.2
(22) Date of filing: 08.01.2007
(51) Int. Cl.: A61K 9/00, A61K 9/16, A61K 9/20, A61K 9/50, A61K 31/223

(54) **PHARMACEUTICAL VALATOGRAST COMPOSITIONS AND PROCESS FOR MANUFACTURING THEM**
PHARMAZEUTISCHE ZUSAMMENSETZUNGEN, ENTHALTEND VALATOGRAST, UND VERFAHREN ZU DEREN HERSTELLUNG
COMPOSITIONS PHARMACEUTIQUES DE VALATOGRAST ET PROCÉDÉ POUR LEUR PRÉPARATION

(30) Priority: 18.01.2006 US 759657 P
(43) Date of publication of application: 15.10.2008
(73) Proprietor: F. Hoffmann-La Roche AG, 4070 Basel (CH)
(72) Inventor: CHATTERJI, Ashish, East Brunswick, New Jersey 08816 (US); SANDHU, Harpreet K., West Orange, New Jersey 07052 (US); SHAH, Navnit Hargovindas, Clifton, New Jersey 07012 (US)
(74) Representative: Niizuma, Yo
(86) International application number: PCT/EP2007/050144
(87) International publication number: WO 2007/082809

(56) References cited:
- WO-A-2006/066780
- WO-A-2006/081986
- WO-A2-2005/051350

## Description

The present invention relates to a novel formulation having as an active ingredient the hydrochloride salt of N-(2-chloro-6-methylbenzoyl)-4-[(2,6-dichlorobenzoyl)amino]-L-phenylalanine-2-(diethylamino)ethyl ester hereinafter referred to as R 411. R411 has activity in the treatment of asthma.

R 411 is available in crystalline form and has pH-dependent physicochemical properties in the physiological range i.e. solubility, stability and permeability.

In addition to pH effect, solubility of R 411 also depends on the type and concentration of counter-ion.

R 411, which has an ester linkage, is also prone to alkaline hydrolysis (at pH>6). The pH-dependent physico-chemical properties and limited permeability (58x10⁻⁶ cm²/sec) result in low and variable oral bioavailability of this compound. Bioavailability in various animal species ranges from 6% (in monkeys) to 20% (in rats and dogs).

Weak bases with pH-dependent physicochemical properties present unique challenges to the formulation scientist. For drugs with dissolution rate limited solubility and bioavailability it becomes a significant challenge. The general approaches use to improve the bioavailability includes reducing the particle size of the drug, use of cosolvents or complexing agents, dispersing the drug in hydrophilic matrices, using lipid based drug delivery systems such as self-emulsifying drug delivery systems, microemulsions, micellar systems and solid dispersion and molecular dispersion and has been source of several literature articles and patents, e.g., Choi et al.,Drug Dev. Ind. Pharm., vol 29(10), 1085-1094, 2003; Yueksel et al., Eur. J. Pharm. and Biopharm., vol 56(3), 453-459, 2003 and US Patent No. 6,632,455.

The bioavailability challenge presented by R411 was not only due to the low solubility but also limited stability in the gastrointestinal fluids. Therefore, requiring formulation approaches which will circumvent both problems. Use of poloxamer to improve the bioavailability based on improved solubility has been documented in the literature (Reddy et al., J. Pharm. Sci, vol 65, 115-118 and 1753-1758, 1976 and Geneidi et al., Pharm. Ind., 42, 315-1980). However, there is no mention in the literature that poloxamer can improve the bioavailability despite the slower dissolution. Furthermore, there is no mention of its effect on the chemical stability of the drug or improving the food effect profile of the drug. These findings form the basis of our invention.

To further improve the stability of the moisture sensitive drugs using internal desiccants is disclosed. The sorption-desorption isotherms for lactose anhydrous indicate that this form of lactose has tendency to absorb moisture at higher humidity. Due to this moisture attraction, lactose anhydrous is not viewed as a favorable excipient for formulating moisture sensitive drugs (Patel et al., Int. J. Pharm.,vol 264, 35-43, Is:1-2, 2003 and Eur, J. Pharm. And Biopharm., vol.46, 177-182, Is:2, 1998). Contrary to the literature data, the inclusion of lactose anhydrous in R411 formulation proved to be beneficial for moisture-mediated instability.

Use of ProSolv as a compression aid is well established. Similarly its superiority over the physical mixture of its individual components, microcrystalline cellulose and colloidal silicone dioxide is also established (Drug Dev. Ind. Pharm.,vol 30, 103-109, Is:1, 2004). However its ability to influence the dissolution stability especially in a favorable way is a surprising finding and is considered as further enhancement in the formulation.

Lastly, the use of processing method is also included. The R411 poloxamer mixtures can be processed by several means. However to get the maximum advantage from bioavailability perspective, an intimate mixing by methods such as melt granulation and melt extrusion is preferred. The utility of several of these techniques has been established in the literature. Their superiority over other methods of granulation for moisture sensitive products is also documented (Passerini et al., Eur. J. Pharm. Sci., vol 15, 71-78, Is:1, 2002). However, the literature limits its advantage only to the initial chemical stability whereas in the case of R411 formulation its advantage is observed during the prolong period of time in the form of dissolution stability which is non-obvious.

The present invention provides a pharmaceutical composition, in oral dosage form, of R411 with improved pharmacokinetic performance, i.e., enhanced bioavailability and reduced food effect. The composition comprises from 50 mg to 400 mg of R411 in a unit dosage together with poloxamer 188 (Lutrol F68®) manufactured by melt granulation or melt extrusion methods. The use of secondary excipients such as ProSolv SMCC50® and Lactose Anhydrous to get aided benefit in the stability is also disclosed.

The subject invention provides a pharmaceutical composition for orally administering R411 which comprises, based on the total weight of the composition, from about 50 mg to 400 mg of R411, and on a % by weight basis from about 5 to 40 % of poloxamer 188, from about 1 to 20% ProSolv SMCC50® and from about 20 to 60% of Lactose Anhydrous.

A preferred composition comprises from 300-400 mg of R411, from about 10 to 25% of poloxamer 188, from about 2 to 4% ProSolv SMCC50® and from about 30 to 50% of Lactose Anhydrous.

A most preferred composition with regards to key excipients is shown below:

| | |
|---|---|
| R411 | 318mg |
| Poloxamer 188 | 136mg |
| Lactose Anhydrous | 228mg |
| ProSolv SMCC50^{®} | 40mg |

Other excipients such as disintegrants and lubricants and fillers can be added as needed to improve the manufacturing and/or performance of the dosage form which are known in the prior art. Poloxamer 188 (Lutrol F68^{®}) is a block copolymer of ethylene oxide and propylene oxide and is listed in the NF monograph as poloxamer 188. Poloxamers are available in wide range of molecular weights, melting points and hydrophilicity and are commonly used in the pharmaceutical formulations as wetting agents to improve the bioavailability . They are supplied by BASF (NJ, USA). The Lutrol F68^{®} used in this invention has molecular weight in the range of 8400 daltons, melting point of 52°-54°C and HLB (hydrophilic-lipophilic balance) of 18-29 and the average particle size ranging from 1 micron to 500 microns. Its utility with R411 in the formulation presents a unique situation as it provides a slow dissolving matrix than the conventional tablet and yet provides higher bioavailability for a drug that has unfavorable solubility and stability in small-intestine. Furthermore, this pharmaceutical composition also minimizes the effect of food on the pharmacokinetic performance of the product. The preferred physical form of poloxamer 188 is fine particle material to achieve intimate mixing. The other nonionic surfactants of secondary choice include Vitamin E TPGS (Eastman Kodak), Gelucire 44/14, Gelucire 50/13 (Gattefosse, NJ), Solutol HS15, poloxamer 407, poloxmaer 338, Lutrol F77, Cremophor RH40 (BASF, NJ), sucrose dipalmitate and sucrose distearate (Croda, NJ).

Lactose anhydrous is supplied by Cary Biosciences (IL, USA) or DMV International (Netherlands). It is a commonly used as bulking agent, compression aid and carrier for pharmaceutical actives. Due to its hygroscopic nature it is not recommended for use with moisture sensitive material, however in this particular case its hygroscopicity imparted the improved dissolution stability as it serves as a moisture scavenger.

ProSolv SMCC^{®} is a co-processed excipient containing 2% colloidal silicon dioxide and 98% microcrystalline cellulose. The individual excipients in the formulation meet the USP/NF requirements. It is supplied by JRS and is available in two grades; ProSolv SMCC50^{®} and ProSoIvSMCC90^{®} related to the different particle size of microcrystalline cellulose i.e 50 microns and 90 microns, respectively. ProSolv is used in the pharmaceutical preparation as compression aid. Its utility in the R411 formulation is not only as compression aid but it also helps improve the dissolution stability of the product. This is a surprising finding as all the literature data shows ProSolv to be superior in compression characteristics however, there is no mention of the improvements in the physical stability by the use of co-processed excipients rather than its individual components.

The polymeric matrix core formulation may further contain other pharmaceutically acceptable excipients such as binders, fillers, stabilizers, compression aids, lubricants, granulation aids, flow aids, and the like. The membrane coating may further contain other coating excipients such as opacifiers, pigments, colourants and the like. The choice of such materials and the amounts to be utilized are considered to be within the art. In order to minimize hardening and rupture of the membrane coating, it is often desirable to utilize a plasticizer in combination with the polymeric coating material. Examples of plasticizers that can be used in accordance with the invention include: triacetin, propylene glycol, polyethylene glycol having a molecular weight of about 200 to about 1,000, dibutyl phthalate, dibutyl sebacate, triethyl citrate, vegetable and mineral oils, fatty acids, fatty acid glycerides of C₆, -C₁₈ fatty acids, and the like.

The core element prepared in accordance with the present invention can be manufactured on conventional tableting equipment, capsule filling equipment or molding equipment.

The invention is further illustrated but not limited to the following examples:

### Example 1

### N-(2-chloro-6-methylbenzoyl)-4-[(2,6-dichlorobenzoyl)amino]-L-phenylalanine-2-(diethylamino)ethyl ester hydrochloride(R411)

The following reaction scheme and steps produce the titled product:

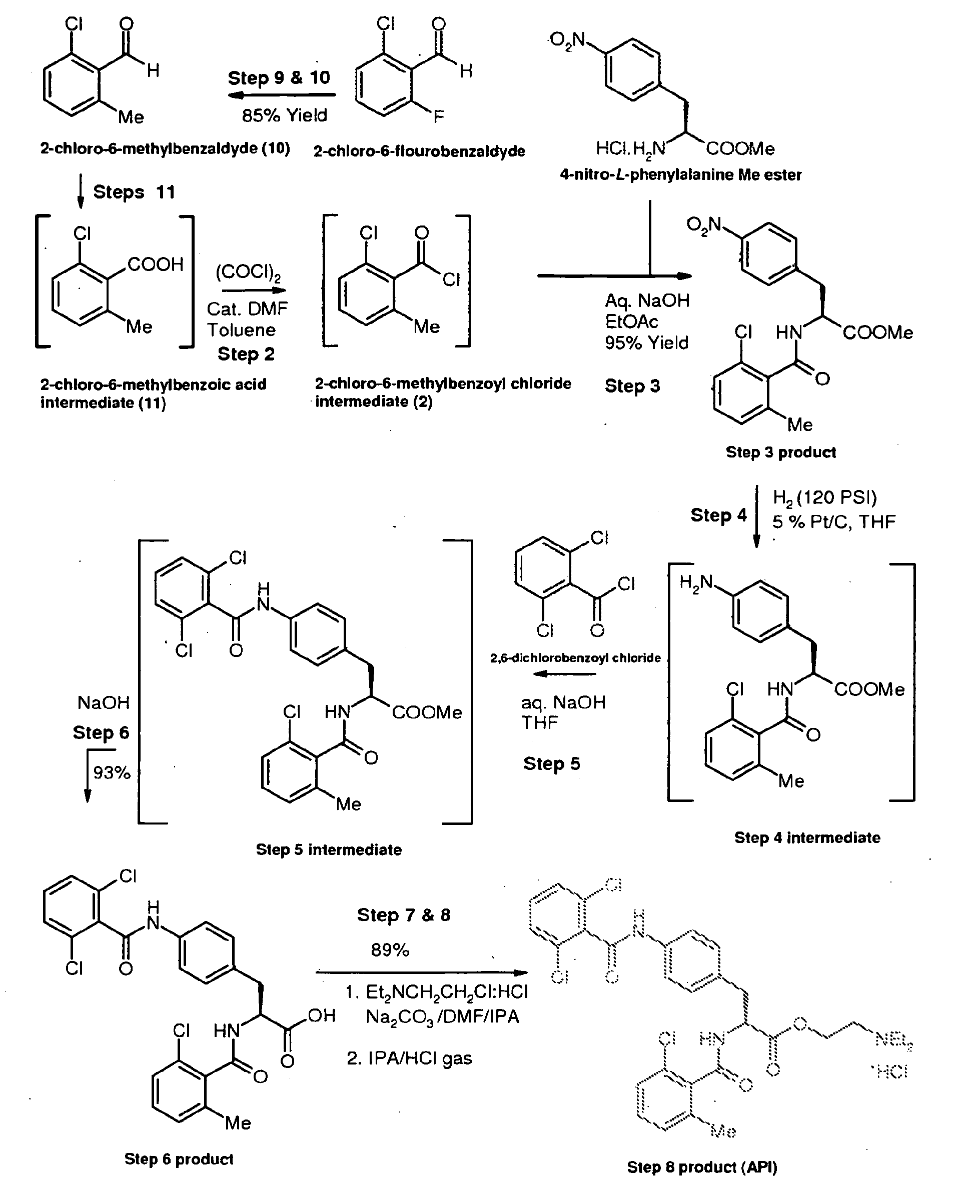

### Preparation of Step 10 product

2-Chloro-6-fluorobenzaldehyde was reacted with n-butylamine in toluene at reflux to prepare the corresponding imine intermediate. After concentration by distillation, reaction mixture is diluted with THF. Metal-halogen exchange of the chloro group was achieved by treating the imine intermediate with 1.4 equivalents of MeMgCl in THF. Imine group was hydrolyzed with dilute acid and the 2-chloro-6-methylbenzaldehyde **(10)** is extracted with hexanes and the step product is purified via vacuum distillation.

### Preparation of Step 3 Product

The 2-chloro-6-methylbenzaldehyde **(10)** is oxidized to 2-chloro-6-methylbenzoic acid intermediate **(11)** in acetonitrile using DMSO/NaClO₂. The excess oxidant is neutralized and the desired acid is extracted with toluene. The trace amount of water in the reaction mixture is removed by partial distillation of toluene. The toluene solution containing the 2-chloro-6-methylbenzoic acid intermediate **(11)** is directly carried into Step 2 reaction. Oxalyl chloride is charged to the toluene solution of 2-chloro-6-methylbenzoic acid **(11)** in presence of a catalytic amount of N,N-dimethylformamide (DMF). After concentration by distillation, water is added to quench the excess oxalyl chloride. The resulting solution of 2-chloro-6-methylbenzoyl chloride intermediate **(2)** is combined with 4-nitro-*L*-ohenylaianine methyl ester hydrochloride in ethyl acetate and aqueous sodium hydroxide. The reaction is completed by the addition of a saturated sodium bicarbonate solution. After completion of the reaction, the batch may be filtered using diatomaceous earth. The layers are separated and the organic phase is washed with water. Ethyl acetate is replaced by toluene by distillation. After cooling and diluting the mixture with hexanes, the Step **3** product is crystallized, collected by filtration, washed with hexanes and dried.

### Preparation of Step 6 Product

The Step **(3)** product was hydrogenated over sulfided Pt/C catalyst in tetrahydrofuran (THF) with heating to give Step **4** intermediate. After removal of the catalyst by filtration the Step **4** intermediate THF solution was cooled, combined with 2,6-dichlorobenzoyl chloride and aqueous sodium hydroxide solution. After completion of the reaction, the Step 5 intermediate is treated with an additional portion of aqueous sodium hydroxide, resulting in the hydrolysis of the methyl ester. The mixture is diluted with isopropyl acetate and acidified with hydrochloric acid. The aqueous layer is separated and the organic layer is washed with water. After replacing the THF and concentration of the isopropyl acetate by distillation, the Step **6** product is crystallized, collected by filtration, washed and dried. This material is a solvate with isopropyl acetate.

### Preparation of Step 8 Product

A mixture of Step **6** product, 2-(diethylamino)ethyl chloride hydrochloride (1 equivalent) and sodium carbonate in DMF is heated to ca. 45°C. After completion of the reaction, the mixture is cooled and combined with isopropyl acetate and aqueous ammonium chloride solution. The aqueous phase is separated and discarded and the organic layer is washed with DMF and water. The batch is diluted with isopropyl acetate and concentrated under vacuum to give a solution of free amine. The batch is tested for water content. The batch is heated, followed by subsurface addition of gaseous hydrogen chloride diluted with nitrogen gas to precipitate the desired hydrochloride salt of the end product(**8**). The batch is cooled, collected by filtration, washed with isopropyl acetate and dried. The end product may be delumped or milled.

### Example 2

### Formulations made by wet granulation method

The R411 drug product can be manufactured by conventional wet granulation method. A typical composition and method of manufacture are illustrated below. The weighed quantities of R411, poloxamer 188, crospovidone, povidone K30, mannitol and fumaric acid are placed in high shear granulator. The contents are mixed with impeller and cross screw set at medium speed for about 2 minutes. The required amount of purified water is added by spraying the water through liquid feed port while mixing the contents. The mixing continues until target granulation end point based on the power consumption reading is achieved. The obtained granules are delumped by Fitz milling at slow speed. The delumped granules are dried at ∼35°C by fluid bed drying until targeted moisture content is reached. The dried granule sare milled by Fitz mill to achieve uniform size granules for further processing. These granules are mixed with external excipients such as crospovidone, talc and magnesium stearate by blending in appropriate blender. This powder mass is compressed into tablets using appropriate tablet compression machine. The compressed tablets are coated with the film-coat shown in the formula by using appropriate tablet coating equipment. Although wet granulation is feasible for this product, it is not feasible due to potential stability concerns and processing challenges. The aqueous wet granulation of this product presented significant manufacturing challenges due to narrow granulation end point. Inadequate control of granulation process causes problems with compaction and dissolution profiles.

| **Wet granulated** | |
|---|---|
| **Ingredient** | **mg** |
| R 411 | 318.00 |
| Poloxamer 188 | 114.00 |
| Povidone K30 | 6.00 |
| Crospovidone (intragranular) | 6.00 |
| Mannitol | 0.00 |
| Fumaric acid | 30.00 |
| Crospovidone (extragranular) | 6.00 |
| Mannitol | 93.00 |
| Talc | 18.00 |
| Mg. Stearate | 9.00 |
| **Total kernel** | **600.00** |

| **Film Coat** | |
|---|---|
| Hypromellose coating system (with talc, colorant and opacifier) | 17.00 |
| Aquacoat ECD30 | 2.18 |
| Triacetin | 0.82 |
| **Total** | **620** |

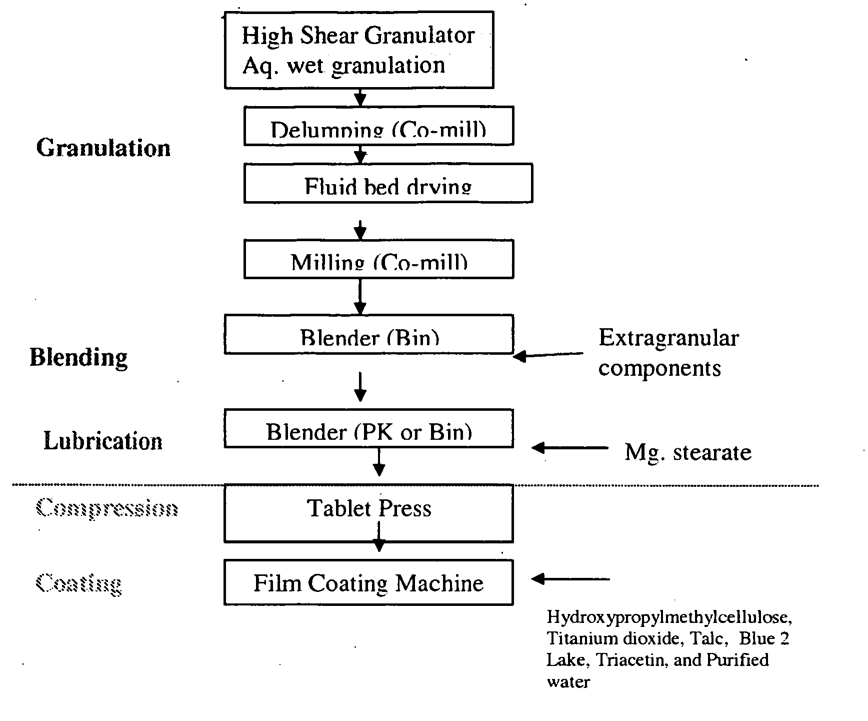

### Example 3

### Formulations made by hot melt granulation

The hot melt granulation for this product takes advantage of low melting point of poloxmaer 188 (52°-54°C). A typical composition is shown in the table and the manufacturing process is presented in the schematic. The manufacturing process consists of high shear granulation, fluid bed cooling, milling, blending, compression and coating. The weighed quantities of R411, poloxmaer 188 and crospovidone (intragranular) are placed in jacketed high shear granulator. The powder is mixed with impeller and chopper at medium speed while the temperature of the jacket is increased by circulating hot water at 60°C. The powder is intermittently mixed until the bed temperature reaches 50°C. The continuous mixing is started as the bed temperature reaches 50°C to assure uniform melting of the poloxamer and uniform granulation. The mixing is continued until targeted granulation end point is achieved with regards to power consumption. The granules are delumped by passing through Fitz mill. The delumped granules are cooled in fluid bed at ambient conditions. The cooled granules are milled through Fitz mill to achieve uniform granules size distribution. The milled granules are mixed with external excipients such as crospovidone (extragranular), fumaric acid, ProSolv, talc, lactose anhydrous and magnesium stearate in a suitable blender. The mixed granules are compressed into kernels using appropriate tablet compression machines. The comressed tablet are film-coated using appropriate film-coat in vented coating pans. Alternatively, the granules can be filled into hard gelatin capsules as well.

| **Hot melt granulated** | |
|---|---|
| **Ingredient** | **mg** |
| R 411 | 318.00 |
| Crospovidone (intragranular) | 8.00 |
| Poloxamer 188 | 136.00 |
| Lactose anhydrous | 228.00 |
| Fumaric acid | 30.00 |
| Talc | 24.00 |
| Crospovidone (extragranular) | 8.00 |
| ProSolv SMCC50^{®} | 40.00 |
| Mg. Stearate | 8.00 |
| | |
| **Total kernel** | **800.00** |

| **Film Coat** | |
|---|---|
| Hypromellose Coating system | |
| (with opacifier, colorant and talc) | 17.00 |
| Aquacoat ECD30 | 2.18 |
| Triacetin | 0.82 |
| **Total** | **820** |

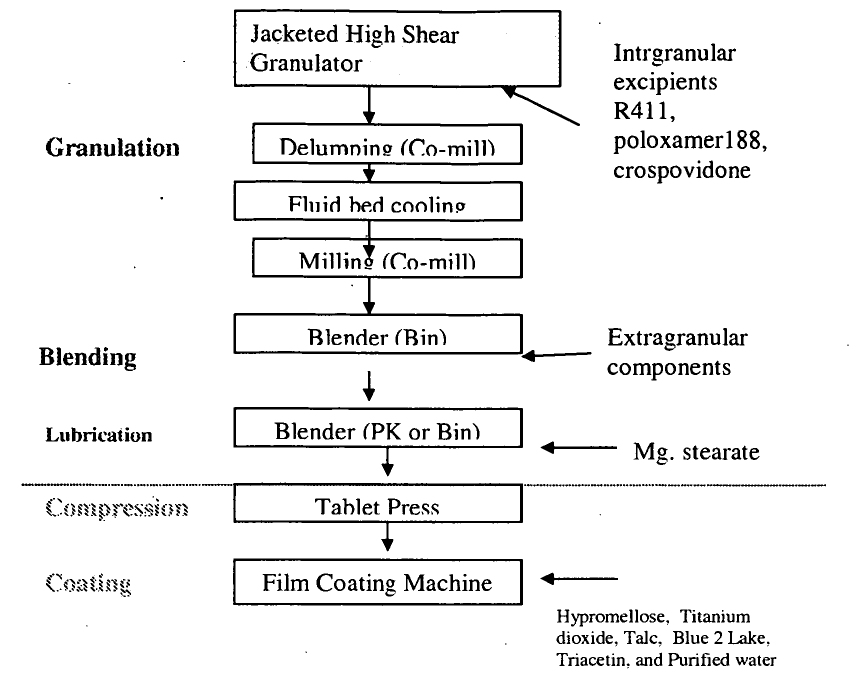

The hot melt granulation process utilizes the melting behavior of poloxamer 188 at 52-54°C to affect the granulation and has provided much superior control of the granulation process. The consistent granulation provides more robust downstream processing and yield reproducible dissolution. Therefore, the hot melt granulation as opposed to conventional aqueous wet granulation provides a more robust product.

### Example 4

### Formulations manufactured by hot melt extrusion method

The granulation of R411 can be achieved by hot melt extrusion process. This is the most preferred method as it provides the most intimate mixing of R411 with poloxamer 188 resulting in more uniform and robust drug product. Since hot melt extrusion process is continuous process, it also provides added advantage in scale-up of the drug product. A typical pharmaceutical composition is provided in the Table with manufacturing process as schematic.

The R411 and poloxamer 188 are mixed together in appropriate blender (bin or twin shell). The mixture is fed into the appropriate extruder (Leistritz twin screw extruder) using gear operated feeder at constant rate (10-20 g/minute) while the screw rotation is maintained at 100-200 rpm. The twin screw extruder is equipped with appropriate screw geometry and the barrel temperatures (for 8 barrels of the Micro-1 unit) are maintained at 30, 40, 60, 70,70,70,70, 70°C. The extrudates are collected from the exit port using 3 mm die and are air cooled on a conveyer belt. The collected extrudates are milled through Fitz mill using appropriate conditions (such as 2 mm screen at medium speed). The granules are blended with external excipients in appropriate blender. The final blend is compressed into tablets using tablet compression machine. The kernels can be coated using appropriate film-coat in the vented coating pans.

| **Hot Melt Extruded** | |
|---|---|
| **Component** | **Amount (mg)** |
| R411 | 318.00 |
| Poloxamer 188 | 170.00 |
| Lactose anhydrous | 100.00 |
| Crospovidone | 25.00 |
| Fumaric acid | 30.00 |
| ProSolv SMCC50^{®} | 40.00 |
| Talc | 15.00 |
| Magnesium stearate | 10.00 |
| Total Kernel Weight | 710.00 |
| Film-coat | 20.00 |
| **Total tablet weight** | **730.00** |

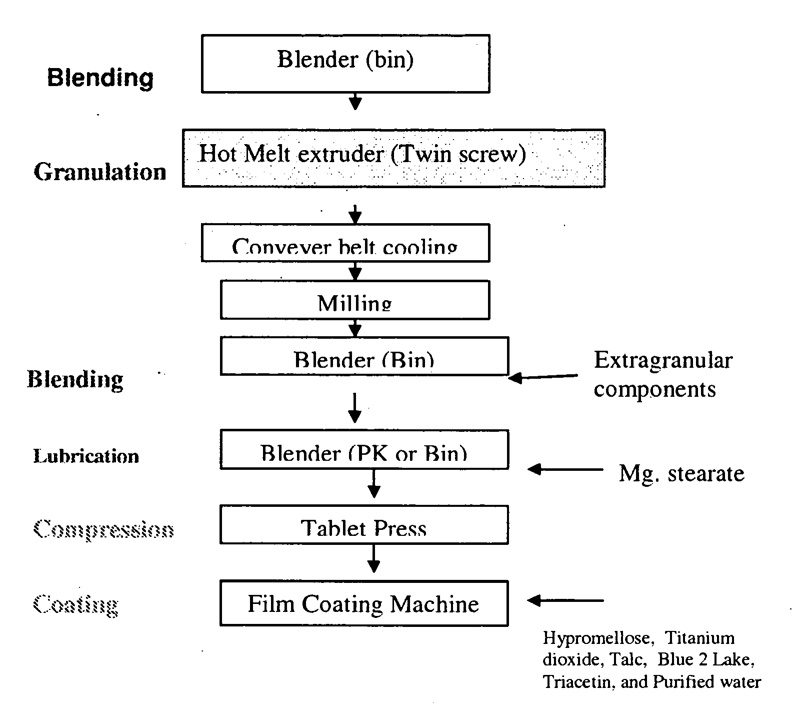

### Example 5

### Formulations made by dry granulation method (slugging or roller compaction)

An example illustrating the drug product manufacturing by dry granulation metjod is presented in this section. A typical pharmaceutical composition is provided in the Table below. The R411, poloxamer 188, ProSolv SMCC50 and portion of magnesium stearate are mixed together in appropriate blender (bin or twin shell). In the case of slugging method, this powder mass is compressed into flat-faced tablets (1" diameter) of approximately 10-15 SCU hardness. For roller compaction, the powder mass is compressed into ribbons by compressing between the moving rollers at desired pressure. The ribbons or the slugs are then milled using appropriate mill (Fitz or Co-mill) to obtain the uniform granules with average granule size in the range of 100-200 microns. The granules are blended with external excipients in appropriate blender. The final blend is compressed into tablets using tablet compression machine. The kernels can be coated using appropriate film-coat in the vented coating pans.

| **Granulation Method** | |
|---|---|
| **Component** | **% w/w** |
| R411 | 40.0 |
| Poloxamer 188 | 15.0 |
| Lactose anhydrous | 17.0 |
| Crospovidone | 4.0 |
| Fumaric acid | 4.0 |
| ProSolv SMCC50^{®} | 14.5 |
| Talc | 2.0 |
| Magnesium stearate | 1.5 |
| Film-coat | 2.0 |

### Example 6

### Formulations containing poloxamer 188 in hard gelatin capsules

The granules obtained by any method as shown in previous examples such as wet granulation, melt granulation, melt extrusion or roller compaction are mixed with external excipients (as needed) and filled into the appropriate size hard shell capsules. A simple example is shown below where R411, poloxamer 188, crospovidone and povidone are granulated by wet granulation method. The granules are dried in fluid bed dryer, milled and mixed with external excipients such as magnesium stearate. The final blend is then filled into hard gelatin capsules.

| **Component** | **Amount (mg)** | **Amount (mg)** |
|---|---|---|
| | 75 mg per capsule | 300 mg per capsule |
| R411 | 79.50 | 318.00 |
| Poloxamer 188 (milled) | 28.80 | 115.20 |
| Crospovidone | 2.28 | 9.12 |
| Povidone K30 | 2.28 | 9.12 |
| Magnesium stearate | 1.14 | 4.56 |
| Total Fill Weight | 114.00 | 456.00 |
| Hard gelatin capsules | 37.80 | 104.00 |
| **Total capsule weight** | **151.80** | **560.00** |

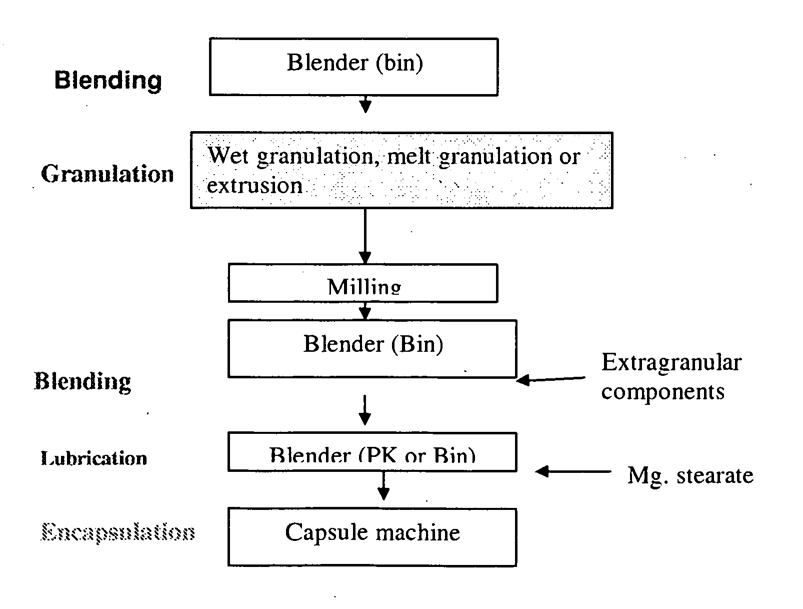

## Claims

1. A composition in a unit dosage form comprising
a) from about 50 mg to 400 mg of N-(2-chloro-6-methylbenzoyl)-4-[(2,6-dichlorobenzoyl)amino]-L-phenylalanine-2-(diethylamino)ethylester hydrochloride and on a % by weight basis b) from about 5 to 40 % of poloxamer 188, c) from about 0 to 20% ProSolv SMCC50® and d) from about 0% to 60% of Lactose Anhydrous.

2. The composition of claim 1 which comprises
a) from 300-400 mg of N-(2-chloro-6-methylbenzoyl)-4-[(2,6-dichlorobenzoyl)amino]-L-phenylaianine-2-(diethylamino)ethylester hydrochloride , b) from about 10 to 25% of poloxamer 188, c) from about 2 to 4% ProSolv SMCC50® and d) from about 30 to 50% of Lactose Anhydrous.

3. The composition of claim 1 wherein said composition is manufactured by hot melt granulation process or hot melt extrusion process.

4. A film coated tablet having a composition which comprises
| | |
|---|---|
| N-(2-chloro-6-methylbenzoyl)-4-[(2,6-dichlorobenzoyl)amino]-L-phenylalanine-2-(diethylamino)ethyl ester | 318.00mg |
| Poloxamer 188 | 114.00mg |
| Povidone K30 | 6.00mg |
| Crospovidone (intragranular) | 6.00mg |
| | |
| Fumaric acid | 30.00mg |
| Crospovidone (extragranular) | 6.00mg |
| Mannitol | 93.00mg |
| Talc | 18.00mg |
| Mg. Stearate | 9.00mg |
| **Total kernel** | **600.00mg** |

5. A film coated tablet having a composition which comprises
| | |
|---|---|
| N-(2-chloro-6-methylbenzoyl)-4-[(2,6-dichlorobenzoyl)amino]-L-phenylalanine-2(diethylamino)ethyl ester | 318.00mg |
| Crospovidone (intragranular) | 8.00mg |
| Poloxamer 188 | 136.00mg |
| Lactose anhydrous | 228.00mg |
| Fumaric acid | 30.00mg |
| Talc | 24.00mg |
| Crospovidone (extragranular) | 8.00mg |
| ProSolv SMCC50^{®} | 40.00mg |
| Mg. Stearate | 8.00mg |
| **Total kernel** | **800.00mg** |

6. A composition comprising
| | |
|---|---|
| N-(2-chloro-6-methylbenzoyl)-4-[(2,6-dichlorobenzoyl)amino]-L-phenylalanine-2-(diethylamino)ethyl ester | 318.00mg |
| Poloxamer 188 | 170.00mg |
| Lactose anhydrous | 100.00mg |
| Crospovidone | 25.00mg |
| Fumaric acid | 30.00mg |
| ProSolv SMCC50^{®} | 40.00mg |
| Talc | 15.00mg |
| Magnesium stearate | 10.00mg |
| Total Kernel Weight | 710.00mg |
| Film-coat | 20.00mg |
| **Total tablet weight** | **730.00mg** |

7. A composition comprising
| | |
|---|---|
| N-(2-chloro-6-methylbenzoyl)-4-[(2,6-dichlorobenzoyl)amino]-L-phenylalanine-2(diethylamino)ethyl ester | 79.50mg |
| Poloxamer 188 (milled) | 28.80mg |
| Crospovidone | 2.28mg |
| Povidone K30 | 2.28mg |
| Magnesium stearate | 1.14mg |
| Total Fill Weight | 114.00mg |
| Hard gelatin capsules | 37.80mg |
| | |
| **Total capsule weight** | **151.80mg** |

8. A composition comprising
| | |
|---|---|
| N-(2-chloro-6-methylbenzoyl)-4-[(2,6-dichlorobenzoyl)amino]-L-phenylalanine-2(diethylamino)ethyl ester | 318.00mg |
| Poloxamer 188 (milled) | 115.20mg |
| Crospovidone | 9.12mg |
| Povidone K30 | 9.12mg |
| Magnesium stearate | 4.56mg |
| Total Fill Weight | 456.00mg |
| Hard gelatin capsules | 104.00mg |
| **Total capsule weight** | **560.00mg** |

## Patentansprüche

1. Zusamtnensetzung in einer Einzeldosisform, umfassend
a) etwa 50 mg bis 400 mg N-(2-Chlor-6-methylbenzoyl)-4-[(2,6-dichlorbenzoyl)amino]-L-phenylalanin-2-(diethylamino)ethylesterhydrochlorid und auf einer Gew.-%-Basis b) etwa 5 bis 40 % Poloxamer 188, c) etwa 0 bis 20 % ProSolv SMCC50® und d) etwa 0% bis 60% wasserfreie Lactose.

2. Zusammensetzung gemäß Anspruch 1, umfassend:
a) 300 bis 400 mg N-(2-Chlor-6-methylbenzoyl)-4-[(2,6-dichlorbenzyl)amino]-L-phenylalanin-2-(diethylarcino)ethylesterhydrochlorid, b) etwa 10 bis 25% Poloxamer 188, c) etwa 2 bis 4% ProSolv SMCC50® und d) etwa 30 bis 50% wasserfreie Lactose.

3. Zusammensetzung gemäß Anspruch 1, wobei die Zusammensetzung durch ein Heißschmelzgranulierungsverfahren oder ein Heißschmelzextrusionsverfahren hergestellt wird.

4. Film-Tablette, welche eine Zusammensetzung aufweist, umfassend
| | |
|---|---|
| N-(2-Chlor-6-methylbenzoyl)-4-[(2,6-dichlorbenzoyl)amino]-L-phenyl-alanin-2-(diethylan-iino)ethylester | 318,00 mg |
| Poloxamer 188 | 114,00 mg |
| Povidon K30 | 6,00 mg |
| Crospovidon (intragranulär) | 6,00 mg |
| Fumarsäure | 30,00 mg |
| Crospovidon (extragranulär) | 6,00 mg |
| Mannit | 93,00 mg |
| Talkum | 18,00 mg |
| Magnesiumstearat | 9,00 mg |
| **Gesamtkern** | **600,00 mg** |

5. Film-Tablette, welche eine Zusammense2zung aufweist, umfassend
| | |
|---|---|
| N-(2-Chlor-6-methylbenzoyl)-4-[(2,6-dichlorbenzoyl)amino]-L-phenyl-alanin-2-(diethylamino)ethylester | 318,00 mg |
| Crospovidon (intragranulär) | 8,00 mg |
| Poloxamer 188 | 136,00 mg |
| wasserfreie Lactose | 228,00 mg |
| Fumarsäure | 30,00 mg |
| Talkum | 24,00 mg |
| Crospovidon (extragranulär) | 8,00 mg |
| ProSolv SMCC50® | 40,00 mg |
| Magnesiumstearat | 8,00 mg |
| **Gesamtkern** | **800,00 mg** |

6. Zusammensetzung, umfassend
| | |
|---|---|
| N-(2-Chlor-6-methylbenzoyl)-4-[(2,6-dichloxbenzoyl)axrxino]-L-phenyl-alanin-2-(diethylamino)ethylester | 318,00 mg |
| Poloxamer 188 | 170,00 mg |
| wasserfreie Lactose | 100,00 mg |
| Crospovidon | 25,00 mg |
| Fumarsäure | 30,00 mg |
| PxoSolv SMCC50® | 40,00 mg |
| Talkum | 15,00 mg |
| Magnesiumstearat | 10,00 mg |
| Gewicht des Gesamtkems | 710,00 mg |
| Filmbeschichtung | 20,00 mg |
| **Gesamtgewicht der Tablette** | **730,00 mg** |

7. Zusammensetzung, umfassend
| | |
|---|---|
| N-(2-Chlor-6-methylbenzoyl)-4-[(2,6-dichlorbenzoyl)amino]-L-phenyl-alanin-2-(diethylamino)ethylester | 79,50 mg |
| Poloxamer 188 (gemahlen) | 28,80 mg |
| Crospovidon | 2,28 mg |
| Povidon K30 | 2,28 mg |
| Magnesiumstearat | 1,14mg |
| Gesamtgewicht der Füllung | 114,00 mg |
| Hartgelatinekapseln | 37,80 mg |
| **Gesamtgewicht der Kapsel** | **151,80 mg** |

8. Zusammensetzung, umfassend
| | |
|---|---|
| N-(2-Chlor-6-methylbenzoyl)-4-[(2,6-dichlorbenzoyl)amino]-L-phenyl-alanin-2-(diethylamino)ethylester | 318,00 mg |
| Poloxamer 188 (gemahlen) | 115,20 mg |
| Crospovidon | 9,12 mg |
| Povidon K30 | 9,12 mg |
| Magnesiumstearat | 4,56 mg |
| Gesamtgewicht der Füllung | 456,00 mg |
| Hartgelatinekapseln | 104,00 mg |
| **Gesamtgewicht der Kapsel** | **560,00 mg** |

## Revendications

1. Composition sous une forme posologique unitaire, comprenant
(a) environ 50 mg à 400 mg de chlorhydrate d'ester 2-(diéthylamino)éthylique de N-(2-chloro-6-méthylbenzoyl)-4-[(2,6-dichlorobenzoyl)amino]-L-phénylalanine et, sur une base de pourcentage en poids, b) environ 5 à 40 % de Poloxamère 188, c) environ 0 à 20 % de ProSolv SMCC50^{®} et d) environ 0 % à 60 % de lactose anhydre.

2. Composition suivant la revendication 1, qui comprend
a) 300 à 400 mg de chlorhydrate d'ester 2-(diéthylamino)éthylique de N-(2-chloro-6-méthylbenzoyl)-4-[{2,6-dichlorobenzoyl)amino]-L-phénylalanine, b) environ 10 à 25 % de Poloxamère 188, c) environ 2 à 4 % de ProSolv SMCC50^{®} et d) environ 30 à 50 % de lactose anhydre.

3. Composition suivant la revendication 1, ladite composition étant produite par un procédé de granulation en masse fondue à chaud ou un procédé d'extrusion en masse fondue à chaud.

4. Comprimé enrobé d'un film, ayant une composition qui comprend
| | |
|---|---|
| ester 2-(diéthylamino)éthylique de N-(2-chloro-6-méthylbenzoyl)-4-[(2,6-dichlorobenzoyl)amino]-L-phénylalanine | 318,00 mg |
| Poloxamère 188 | 114,00 mg |
| Povidone K30 | 6,00 mg |
| Crospovidone (intragranulaire) | 6,00 mg |
| | |
| Acide fumarique | 30,00 mg |
| Crospovidone (extragranulaire) | 6,00 mg |
| Mannitol | 93,00 mg |
| Talc | 18,00 mg |
| Stéarate de Mg | 9,00 mg |
| | |
| **Noyau total** | **600,00 mg** |

5. Comprimé enrobé d'un film, ayant une composition qui comprend :
| | |
|---|---|
| ester 2-(diéthylamino)éthylique de N-(2-chloro-6-méthylbenzoyl)-4-[(2,6-dichlorobenzoyl)amino]-L-phénylalanine | 318,00 mg |
| Crospovidone (intragranulaire) | 8,00 mg |
| Poloxamère 188 | 136,00 mg |
| Lactose anhydre | 228,00 mg |
| Acide fumarique | 30,00 mg |
| Talc | 24,00 mg |
| Crospovidone (extragranulaîre) | 80,00 mg |
| ProSolv SMCC50^{®} | 40,00 mg |
| Stéarate de Mg | 9,00 mg |
| | |
| **Noyau total** | **800,00 mg** |

6. Composition comprenant
| | |
|---|---|
| ester 2-(diéthylamino)éthylique de N-(2-chloro-6-méthylbenzoyl)-4-[(2,6-dichlorobenzoyl)amino]-L-phénylalanine | 318,00 mg |
| Poloxamère 188 | 170,00 mg |
| Lactose anhydre | 100,00 mg |
| Crospovidone | 25,00 mg |
| Acide fumarique | 30,00 mg |
| ProSolv SMCC50^{®} | 40,00 mg |
| Talc | 15,00 mg |
| stéarate de magnésium | 10,00 mg |
| Poids du noyau total | 710,00 mg |
| Enrobage constitué d'un film | 20,00 mg |
| | |
| **Poids total du comprimé** | **730,00 mg** |

7. Composition comprenant
| | |
|---|---|
| ester 2-(diéthylamino)éthylique de N-(2-chloro-6-methylbenzoyl)-4-[(2.6-dichlorobenzoyl)amino]-L-phénylalanine | 79,50 mg |
| Poloxamère 188 (broyé) | 28,80 mg |
| Crospovidone | 2,28 mg |
| Povidone K30 | 2,28 mg |
| Stéarate de magnésium | 1,14 mg |
| Poids total du remplissage | 114,00 mg |
| Capsules de gélatine durcie | 37,80 mg |
| | |
| **Poids total de la capsule** | **151,80 mg** |

8. Composition comprenant
| | |
|---|---|
| ester 2-(diéthylamino)éthylique de N-(2-chloro-6-méthylbenzoyl)-4-[(2,6-dichlorobenzoyl)amino]-L-phénylalanine | 318,00 mg |
| Poloxamère 188 (broyé) | 115,20 mg |
| Crospovidone | 9,12 mg |
| Povidone K30 | 9,12 mg |
| Stéarate de magnésium | 4,56 mg |
| Poids total du remplissage | 456,00 mg |
| Capsules de gélatine durcie | 104,00 mg |
| | |
| **Poids total de la capsule** | **560,00 mg** |
